# EUROPEAN PATENT APPLICATION

(11) **EP 4 487 839 A1**
(43) Date of publication of application: **08.01.2025**
(21) Application number: 23216824.5
(22) Date of filing: 14.12.2023
(51) Int. Cl.: A61K 9/20, A61K 31/132

(54) **PHARMACEUTICAL DOSAGE FORM COMPRISING TRIENTINE TETRAHYDROCHLORIDE, AND METHOD FOR PREPARING THE SAME**

(30) Priority: 03.07.2023 IN 202311044471
(71) Applicant: Alfred E. Tiefenbacher (GmbH & Co. KG), 22767 Hamburg (DE)
(72) Inventor: Khan, Baji Hussain, 502 319 Gaddapotharam Village, Kazipally Industrial Area Sangareddy District (IN); Clausen, Ina, 22767 Hamburg (DE); Balivada, Srinivasa Rao, 502 319 Gaddapotharam Village, Kazipally Industrial Area Sangareddy District (IN); Rachakonda, Veereswara Rao, 502 319 Gaddapotharam Village, Kazipally Industrial Area Sangareddy District (IN); Donga, Nani Prasad, 502 319 Gaddapotharam Village, Kazipally Industrial Area Sangareddy District (IN); Kambhampati, Anil Kumar, 502 319 Gaddapotharam Village, Kazipally Industrial Area Sangareddy District (IN); Steiert, Nico, 22767 Hamburg (DE); Joshi, Abhay Ramakant, 502 319 Gaddapotharam Village, Kazipally Industrial Area Sangareddy District (IN)
(74) Representative: Ter Meer Steinmeister & Partner

(57) **Abstract**

The present invention relates to a pharmaceutical dosage form of trientine tetrahydrochloride that has low stickiness and good fluidity, and that can be stably manufactured. The pharmaceutical dosage form comprises crystalline trientine tetrahydrochloride and at least one pharmaceutically acceptable excipient. Moreover, the present invention relates to a method of preparing the pharmaceutical dosage form.

## Description

### Field of the invention

The present invention relates to a pharmaceutical dosage form comprising crystalline trientine tetrahydrochloride, and to a method for preparing the same. In particular, the present invention relates to a pharmaceutical dosage form comprising crystalline trientine tetrahydrochloride that has low stickiness and good flowability, and that can be stably manufactured.

### Background art

Trientine (N,N'-Bis(2-aminoethyl)ethane-1,2-diamine; TETA) is a low molecular weight organic compound that is used in various fields. For medical applications, the hydrochloride salts of trientine have turned out to be particularly useful. Due to their metal chelating properties, hydrochloride salts of trientine have been found to be specifically effective in the treatment of subjects suffering from Wilson's disease, a genetic disorder that is caused by a mutation in the ATP7B gene and is associated with excessive copper accumulation in the liver, in the eyes and in the brain. If untreated, subjects suffering from Wilson's disease typically develop a number of liver disorders or/and neuropsychiatric symptoms, with specific examples of liver disorders including jaundice, portal hypertension and chronic active hepatitis.

Trientine dihydrochloride as approved by the European Medicines Agency (EMA) for the treatment of Wilson's disease in patients intolerant to D-penicillamine therapy is marketed in the European Union by Univar Solutions BV under the tradename Cufence^{®}. Trientine dihydrochloride is a white to pale yellow crystalline hygroscopic powder which exists in three crystalline forms. However, the compound has been shown to be quite sensitive to water and to easily change its crystalline form dependent on the humidity level. Moreover, it was found out that trientine dihydrochloride has insufficient stability at room temperature. Therefore, it is relatively difficult to provide a pharmaceutical dosage form of crystalline trientine dihydrochloride that is stable under ambient conditions over a long time period.

Trientine tetrahydrochloride obtained regulatory approval from the EMA for the treatment of Wilson's disease in patients intolerant to D-penicillamine therapy in 2017. Trientine tetrahydrochloride as marketed in the European Union by GMP-Orphan SA under the tradename Cuprior^{®} is a white, crystalline non-hygroscopic powder which exists in two crystalline forms. In this context, Patent Document 1 discloses that the crystalline form characterized by an XRPD pattern having at least two peaks selected from the peaks at 22.9, 25.4, 25.8, 26.6, 34.6 and 35.3 ± 0.1°2θ (hereinafter also referred to as trientine tetrahydrochloride polymorphic Form B) has both better handling properties and better room temperature stability than the crystalline form characterized by an XRPD pattern having peaks at 21.8, 25.2, 26.9, 28.2 and 35.7 ± 0.1°2θ (hereinafter also referred to as trientine tetrahydrochloride polymorphic Form A).

### Citation list

Patent Document 1: WO 2019/211464 A1

### Summary of the invention

### Problem to be solved

The commercial product Cuprior^{®} is sold in the form of film-coated tablets containing 150 mg of trientine tetrahydrochloride polymorphic Form B. The film-coated tablets consist of a core tablet and a tablet coating, wherein the core tablet comprises mannitol, colloidal anhydrous silica and glycerol dibehenate as mandatory excipients. Surprisingly, it was now discovered that preparation of at least the tablet core of Cuprior^{®} is associated with problems, including a relatively high stickiness and insufficient flowability of the mixture to be tableted. In addition, it has been surprisingly found that trientine tetrahydrochloride polymorphic Form A tends to transform to trientine tetrahydrochloride polymorphic Form B upon pressure. Therefore, pharmaceutical dosage forms comprising a crystalline form of trientine tetrahydrochloride still have room for further improvement, in particular with respect to polymorphic stability, handling properties and polymorphic stability of a mixture underlying the core tablet.

### Means for solving the problem

In order to address the aforementioned deficiencies, the present inventors carried out intense studies and unexpectedly found that the physicochemical properties of a pharmaceutical dosage form of crystalline trientine tetrahydrochloride can be remarkably improved by suitably controlling the average particle diameter of trientine tetrahydrochloride, the type of pharmaceutical acceptable excipients admixed with trientine tetrahydrochloride, or/and the pressure conditions applied during preparation of the pharmaceutical dosage form. In particular, it turned out that the presence of specific excipients comprised by the commercial product Cuprior^{®} directly correlates with increased stickiness of the mixture to be formed into the tablet core, such that (partial) omission thereof can positively influence the characteristics of the formulation. The present invention is defined by the claims.

In a first aspect, the present invention thus relates to a pharmaceutical dosage form as defined in claim 1. The pharmaceutical dosage form comprises crystalline trientine tetrahydrochloride and at least one pharmaceutically acceptable excipient.

In a second aspect, the present invention relates to a process of preparing the pharmaceutical dosage form of the first aspect as defined in claim 13. The preparation process comprises the steps of (i) providing crystalline trientine tetrahydrochloride and at least one pharmaceutically acceptable excipient; (ii) blending the components from step (i) to obtain a mixture; (iii) direct compressing the mixture of step (ii) to obtain a tablet, and (iv) optionally coating the tablet.

Preferred embodiments of the pharmaceutical dosage form and the preparation process of the present invention are described in the subclaims.

### Brief description of the drawings

Fig. 1 shows XRPD patterns of core tablets formed from trientine tetrahydrochloride polymorphic Form A and having a hardness of 200 N, 180 N or 140 N after applying a compression force of 9.8 kN (1 tf) for 2 seconds thereto.
Fig. 2 shows XRPD patterns of core tablets formed from trientine tetrahydrochloride polymorphic Form A and having a hardness of 200 N, 180 N or 150 N after applying a compression force of 29.4 kN (3 tf) for 2 seconds thereto.
Fig. 3 shows XRPD patterns of core tablets formed from trientine tetrahydrochloride polymorphic Form A and having a hardness of 311 N after applying a compression force of 39.2 kN (4 tf) for 2 seconds thereto, and a hardness of 319 N after applying a compression force of 49.0 kN (5 tf) for 2 seconds thereto, respectively.
Fig. 4 shows XRPD patterns of core tablets formed from trientine tetrahydrochloride polymorphic Form A after applying a compression force of 9.8 kN (1 tf) or 49.0 kN (5 tf) for 30 seconds thereto.

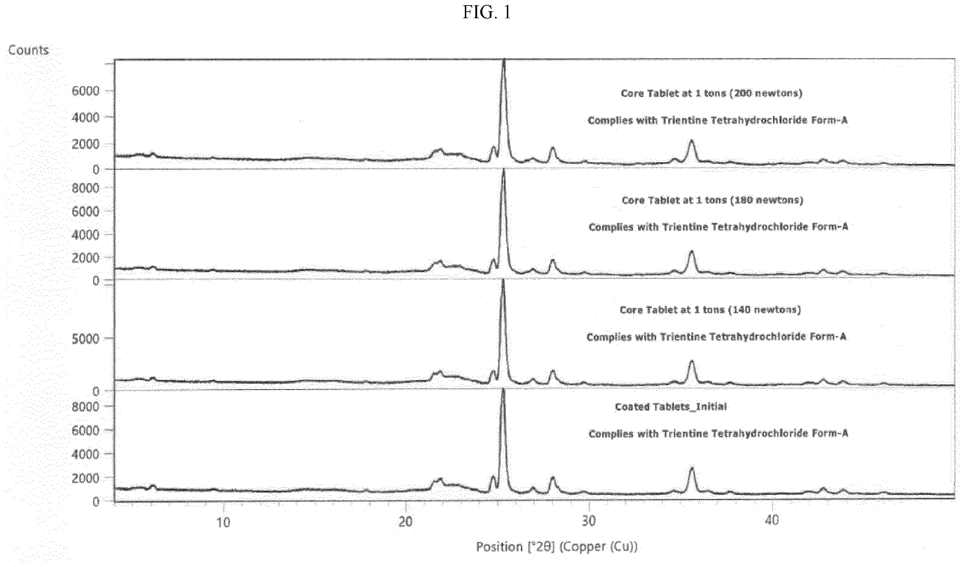

In Fig. 1 to 3, the "coated tablets_initial" have been produced by applying a compression force of 3 tons for 2 seconds. Afterwards, the tablet core has been film coated to obtain a "coated tablet". It is called "initial" because those lab scale tablets represent the "best mode" and are put on stability afterwards. The hardness of said "initial coated tablets" was in the range of 130 to 140 N.

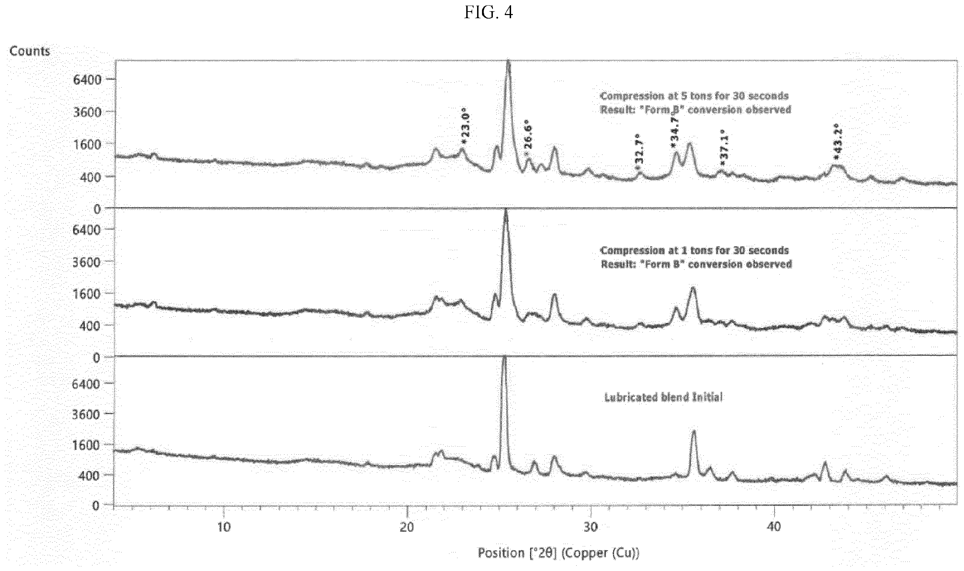

In Fig. 4, "lubricated blend initial" means the blend of the tablet core before any compression which is measured by XRPD. Further, the "lubricated blend" of the upper two XRPDs in Fig. 4 were first compressed to core tablets and then analysed by XRPD.

In Figs. 1 to 4, the term "tons" means the unit "metric ton force" as abbreviated by "tf".

### Detailed description of the invention

The pharmaceutical dosage form of the invention obligatorily comprises crystalline trientine tetrahydrochloride and at least one pharmaceutically acceptable excipient. In this context, the term "pharmaceutical dosage form" as used herein generally designates a liquid, solid or semisolid pharmaceutical formulation which contains at least one active ingredient in combination with at least one pharmaceutically acceptable excipient, and which can be administered to a subject in need thereof by any suitable route of administration. The subject receiving the pharmaceutical dosage form is preferably a human being, and the pharmaceutical dosage form is preferably formulated for oral administration.

In one embodiment, the crystalline trientine tetrahydrochloride comprised by the pharmaceutical dosage form of the invention has a volume-based particle size distribution (PSD) in which the d50 value, the d90 value or/and the d10 value of a powder of the active ingredient is set to a specific range. The term d90 (also D90) means that 90 % of the particles, by volume, have a diameter of or below a given value or range. Similarly, the term d50 (also D50) means that 50% of the particles, by volume, have a diameter of or below a given value or range. The term d10 (also D10) means that 10% of the particles, by volume, have a diameter of or below a given value or range. For example, by adjusting the average particle size (d50) of crystalline trientine tetrahydrochloride to a range of 30 to 600 µm, preferably 45 to 450 µm, it is possible to increase the flowability of a formulation to be tableted and to thereby simplify the overall production process of the pharmaceutical dosage form. A similar technical effect can be achieved by adjusting the d90 value of the powdery active ingredient to a range of 70 to 1000 µm, preferably 100 to 500 µm, or by adjusting the d10 value of the powdery active ingredient to a range of 8 to 500 µm, preferably 15 to 150 µm.

The volume-based particle size distribution of crystalline trientine tetrahydrochloride can be monomodal or bimodal, and is preferably monomodal. Methods for measuring the particle size distribution are well known in the art and include both dry methods and wet methods, with specific examples of such measurement methods including sieve analysis, laser diffraction spectrometry (LDS) and dynamic light scattering (DLS). In one embodiment, the d50 value, the d90 value and the d10 value of crystalline trientine tetrahydrochloride are determined in accordance with a dry method using laser diffraction spectrometry (LDS). In another embodiment, the measurement comprises a wet method in which the active ingredient is dispersed in a suitable dispersing agent such as methanol or ethanol, and the particle size distribution is then measured using a suitable technique such as laser diffraction spectrometry (LD S).

In one embodiment, the crystalline trientine tetrahydrochloride comprised by the pharmaceutical dosage form of the invention is present as trientine tetrahydrochloride polymorphic Form A, i.e. has an XRPD pattern having peaks at 21.8, 25.2, 26.9, 28.2 and 35.7 ± 0.1°2θ. In this context, it is preferred that the crystalline trientine tetrahydrochloride substantially consists of trientine tetrahydrochloride polymorphic Form A, i.e. does not contain any detectable amounts of trientine tetrahydrochloride polymorphic Form B. As used herein, the wording "does not contain any detectable amounts of trientine tetrahydrochloride polymorphic Form B" means that an XRPD spectrum of the pharmaceutical dosage form obtained in accordance with the method described hereinafter does not show any characteristic peaks of trientine tetrahydrochloride polymorphic Form B within the limits of experimental error.

In a preferred embodiment, the pharmaceutical dosage form of the present invention can comprise trientine tetrahydrochloride equivalent to 75 to 300 mg trientine, more preferably equivalent to 150 to 225 mg trientine. In particular, the present pharmaceutical dosage form can comprise trientine tetrahydrochloride equivalent to 75 mg trientine. Alternatively, particularly preferred, the present pharmaceutical dosage form can comprise trientine tetrahydrochloride equivalent to 150 mg trientine. Alternatively, particularly preferred, the present pharmaceutical dosage form can comprise trientine tetrahydrochloride equivalent to 225 mg trientine.

Pharmaceutically acceptable excipients which are suitable for use in the invention are known to a person skilled in the art and comprise, for instance, fillers, lubricants, glidants, anti-sticking agents and disintegrants as disclosed e.g. in US 2006/0045865 A1.

Fillers can be used to increase the bulk volume and weight of a low-dose drug to a limit at which a pharmaceutical dosage can be formed. Fillers may fulfil several requirements, such as being chemically inert, non-hygroscopic and biocompatible. Examples of fillers are microcrystalline cellulose, dextrose, lactose, sucrose, glucose, calcium carbonate, cellulose and others.

The function of lubricants is reported to ensure that tablet formation and ejection can occur with low friction between the solid and the wall. The lubricant is preferably a stearate or fatty acid, more preferably an earth alkali metal stearate, such as magnesium stearate.

Glidants are reported to be substances used to improve the flowability. Examples of glidants are talc, fumed or colloidal silica (for example Aerosil^{®}).

Generally, anti-sticking agents are reported to be substances to prevent the adhesion of the tableting mass to the compression mould. Further anti-sticking agents may increase the brightness of a tablet. The anti-sticking agent can be for example talcum, magnesium stearate, paraffin.

Disintegrants are reported to be compounds which can enhance the ability of the pharmaceutical dosage form to break into smaller fragments when in contact with a liquid, preferably water. Examples of disintegrants are crosslinked carboxymethyl cellulose sodium (croscarmellose sodium), cross-linked polyvinylpyrrolidone (for example Kollidon^{®} Cl), sodium carboxymethyl starch glycolate (for example Explotab^{®}), swelling polysaccharide, for example soy polysaccharide, carrageenan, agar, pectin, starch and derivatives thereof, protein, for example formaldehyde-casein, sodium bicarbonate or mixtures thereof.

Regarding the above-mentioned pharmaceutically acceptable excipients, the application generally refers to "Lexikon der Hilfsstoffe für Pharmazie, Kosmetik und angrenzende Gebiete", edited by H. P. Fiedler, 5th Edition, Editio Cantor Verlag, Aulendorf and earlier editions, and "Handbook of Pharmaceutical Excipients", third edition, edited by Arthur H. Kibbe, American Pharmaceutical Association, Washington, USA, and Pharmaceutical Press, London.

In this regard it is generally noted that due to the nature of pharmaceutical excipients, it cannot be excluded that a certain compound meets the requirements of more than one of the components. However, to enable an unambiguous distinction, it is preferred in the present application that one and the same pharmaceutical compound be only used as one of the compounds. For example, if magnesium stearate is used as lubricant, it is not additionally used as anti-sticking agent, even though magnesium stearate also exhibits a certain anti-sticking effect.

In one embodiment, the at least one pharmaceutical acceptable excipient comprised by the pharmaceutical dosage form of the invention does not include mannitol, or/and does not include glycerol dibehenate. By avoiding the presence of mannitol or/and glycerol dibehenate, it is surprisingly possible to significantly reduce the stickiness of a mixture to be formed into the pharmaceutical dosage form, and to thereby simplify the overall production process of a pharmaceutical dosage form comprising crystalline trientine tetrahydrochloride.

In another embodiment, stickiness of a mixture to be processed to a pharmaceutical dosage of crystalline trientine tetrahydrochloride can be reduced by making the at least one pharmaceutically acceptable excipient to include microcrystalline cellulose (MCC) or/and a C1 to C30 fatty acid, the fatty acid preferably being a C15 to C20 fatty acid such as stearic acid. Particularly good results with respect to reduced stickiness can be obtained by replacing mannitol as included in the core tablet of Cuprior^{®} by microcrystalline cellulose, or/and by replacing glycerol dibehenate as also contained in the tablet core of Cuprior^{®} by a C1 to C30 fatty acid. Simultaneous substitution of mannitol and glycerol behenate by microcrystalline cellulose and a C1 to C30 fatty acid is more preferred.

In one embodiment, microcrystalline cellulose is present in an amount of 10 to 60 wt%, preferably 20 to 50 wt.%, more preferably 25 to 45 wt.%, based on the weight of the dosage form. In case the dosage form is a coated tablet, the weight is based on the tablet with coating.

In one embodiment, the C1 to C30 fatty acid is present in an amount of 1 to 10 wt%, preferably 2 to 8 wt.%, more preferably 3 to 6 wt.%, based on the weight of the dosage form. In case the dosage form is a coated tablet, the weight is based on the tablet with coating.

As explained hereinbefore, the pharmaceutical dosage form of the invention may be present as a liquid, solid or semisolid. Examples of liquid, solid or semisolid pharmaceutical formulations are known to a person skilled in the art and comprise, for instance, capsules, cremes, emulsions, gels, granules, ointments, pastes, powders, solutions, sprays, suppositories, suspensions, syrups, tablets and tinctures, but are not limited thereto. In one preferred embodiment, however, the pharmaceutical dosage form is an optionally coated tablet (such as a film-coated tablet), and more preferably a tablet that comprises a tablet core and at least one tablet coating surrounding the core tablet. In the latter case, crystalline trientine tetrahydrochloride may be included in both the core tablet and in the tablet coating, but is preferably included in the core tablet only.

Preferably, the coating is a film coating. Generally, film coatings that do not affect the release of the active agent(s) and film coatings affecting the release of the active agent(s) can be employed with the tablets according to the invention. The film coatings that do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP), polyvinyl acetate (PVA) and mixtures thereof. More preferred is hydroxypropyl methylcellulose (HPMC). These polymers can have a weight-average molecular weight of 10,000 to 150,000 g/mol.

In a preferred embodiment the film can have a thickness of 2 µm to 150 µm, preferably 10 to 100 µm, more preferably 20 to 60 µm.

When the pharmaceutical dosage form of the invention is formulated as an optionally coated tablet, it is preferred that the tablet has a hardness of less than 300 N, preferably 50 to 280 N, more preferably 100 to 250 N, as determined according to Ph.Eur. 6.0, Chapter 2.9.8. By setting the hardness of the tablet to the aforementioned range, it is surprisingly possible to reliably prevent conversion of trientine tetrahydrochloride polymorphic Form A to trientine tetrahydrochloride polymorphic Form B during preparation of the pharmaceutical dosage form even if the mixture to be tableted is subjected to harsh production conditions.

As an example of harsh production conditions, direct compression of a mixture underlying the pharmaceutical dosage form with a tablet press, such as an eccentric press or a rotary press, at a compression force of more than 39.2 kN (4 tf) or/and at a compression time of more than 30 seconds is mentioned. By suitably varying the thickness of the mixture to be tableted in the tablet press, optionally coated tablets varying in their hardness can be produced without changing the compression force or/and the compression time applied to the mixture.

In addition, the optionally coated tablet preferably can have a friability of less than 5%, particularly preferably less than 2%, especially less than 1%, in particular 0.5 to 0.9%. The friability is determined in accordance with Ph. Eur., 7.7, chapter 2.9.7.

Further, the optionally coated tablets of the invention preferably have a content uniformity, i.e. a content of active agent(s), which lies within the concentration of 90 to 110%, preferably of 95 to 105%, especially preferred of 98 to 102% of the average content of the active agent(s). The "content uniformity" is determined with a test in accordance with Ph. Eur., 6.0, Chapter 2.9.6. According to that test, the content of the active agents of each individual tablet out of 20 tablets must lie between 90 and 110%, preferably between 95 and 105%, especially between 98 and 102% of the average content of the active agents(s). Therefore, the content of the active drugs in each tablet of the invention differs from the average content of the active agent by at most 10%, preferably by at most 5% and especially by at most 2%.

Hardness, friability and content uniformity are determined from an uncoated tablet.

In a second aspect, the present invention relates to a process of preparing the pharmaceutical dosage form of the invention. The pharmaceutical dosage form of the invention can be prepared by a process comprising the steps of (i) providing crystalline trientine tetrahydrochloride and at least one pharmaceutically acceptable excipient; (ii) blending the components from step (i) to obtain a mixture; (iii) direct compressing the mixture of step (ii) to obtain a tablet, and (iv) optionally coating the tablet. In this context, the step (ii) of blending crystalline trientine tetrahydrochloride as defined above with the at least one pharmaceutically acceptable excipient can be carried out in accordance with conventional techniques, e.g. by dry-blending the individual components using a mixing device, such as a free-fall-mixer, so as to provide a mixture which is essentially homogeneous. Blending can be carried out e.g. for 1 minute to 30 minutes, preferably for 2 minutes to less than 10 minutes. It is further preferred that the blend obtained after step (ii) can be sieved using a sieve having a suitable mesh size, preferably with a sieve having a mesh size of 25 to 1000 µm, preferably 50 to 800 µm, especially 100 to 600 µm.

The mixture obtained from the blending step is subsequently subjected to direct compression, and is preferably carried out on a rotary press using dies made of tungsten carbide and punches made of PK3 so as to reduce sticking of the mixture to the production equipment. The compression force applied in the compression step can vary in accordance with needs, but typically lies in the range of from 4.9 kN (0.5 tf) to 39.2 kN (4 tf) for a compression time of less than 30 seconds, preferably 1 to 5 seconds. By controlling the compression force at less than 39.2 kN (4 tf) while applying the compression force for 2 seconds or less, it is possible to substantially lower the risk of trientine tetrahydrochloride polymorphic Form A converting to trientine tetrahydrochloride polymorphic Form B during formation of the tablet.

In a final step, the tablet obtained from the compression step is optionally further provided with a coating. By adding a tablet coating to the (core) tablet, it is possible to better protect crystalline trientine tetrahydrochloride from undesired dissolution or decomposition, and to adjust the release profile of the active ingredient in accordance with needs. Excipients for forming a tablet coating are known to a person skilled in the art and comprise, for instance, binding agents, fillers, lubricants and disintegrators as disclosed e.g. in US 2006/0045865 A1. A specific example of a tablet coating which is suitable for use in the invention comprises a combination of polyvinyl alcohol, talc, titanium dioxide (E 171), glycerol monocaprylocaprate (Type I), iron oxide yellow (E 172) and sodium lauryl sulfate.

### EXAMPLES

The invention will now be explained in more detail based on the following examples which are not intended to limit the scope of the claims.

### Materials and measurement methods

The **particle size distribution** of crystalline trientine tetrahydrochloride was determined using either a dry method or a wet method. In both cases, the particle size distribution was measured by laser diffraction spectrometry (LDS) using a Mastersizer 3000 particle size measuring device (commercially available from Malvern Panalytical Ltd.). In the wet method, a Hydro MV wet dispersion unit (commercially available from Malvern Panalytical Ltd.) was employed, and the method was carried out in accordance with the following procedure using saturated ethanol as the dispersing agent.

### Equipment:

| | |
|---|---|
| Model: | Mastersizer 3000 or equivalent |
| Maker: | Malvern Instruments Ltd or equivalent |
| Accessory Name: | Hydro MV |
| Analysis method: | Wet method |

### Experimental parameters:

### Material properties

| | |
|---|---|
| Particle RI: | 1.52 |
| Particle shape: | Irregular |
| Absorption index: | 0.1 |
| Particle size range: | 0.01 to 3500 µm |

| Dispersant properties | |
|---|---|
| Dispersant RI: | 1.361 |
| Dispersant: | Saturated ethanol |

### Instrument properties

| Analysis model: | General purpose |
|---|---|
| Stirrer speed: | 2000 |
| Obscuration range: | 5 to 15% |
| Measurement Time: | 10 seconds |
| Background Time: | 10 seconds |
| Measurement Repeat: | 3 times |

### Preparation of dispersant (saturated ethanol):

Weigh approximately 4 g of trientine tetrahydrochloride into 1000 mL of ethanol, stir and sonicate for about 15 minutes. Filter the solution through a 0.45 µm PVDF filter and the clear solution is used as dispersant.

### Cleaning of the measurement cell:

Rinse the measurement cell twice with isopropyl alcohol, followed by twice with methanol, followed by 3 times with ethanol. After analysis, rinse the measurement cell twice with ethanol followed by methanol and isopropyl alcohol.

### Measurement procedure:

1. Fill the measurement cell with saturated ethanol in Hydro MV accessory, align the system then measure the background scan
2. Approximately weigh 50 mg of trientine hydrochloride into 100 ml glass beaker, add 30 ml of saturated ethanol sonicate for 30 seconds
3. The sample solution is transferred into the measurement cell until the required obscuration is obtained.
4. Before measurement, it was waited for obscuration to stabilize and the sample was measured 3 times at zero second time intervals.

The measurements are performed for 2 individual preparations and the average result (instrument average) is reported.

The **hardness of core tablets** or film-coated tablets comprising crystalline trientine tetrahydrochloride was determined in accordance with Ph.Eur. 6.0, Chapter 2.9.8.

The conversion of trientine tetrahydrochloride polymorphic Form A to trientine tetrahydrochloride polymorphic Form B was determined using **X-ray powder diffraction (XRPD).**

**XRPD** (transmission mode): For X-Ray Powder Diffraction (XRPD) the sample was placed between two thin polymer films (Kapton foils), after careful grinding. The measurement was performed at room temperature with a PANalytical Empyrean Diffractometer (Cu-Kal = 1.5406 Ä, BBHD optics, position sensitive detector) in transmission mode with rotation of the sample. Data were collected in a two-theta range of 2 to 50°. The tube voltage and current were set to 45 kV and 40 mA, respectively.

Stickiness and flowability of mixtures to be formed into a core tablet by direct compression were visually determined and rated.

### Examples 1 to 6

Six batches of film-coated tablets (batch size: 500 to 1500 tablets) comprising crystalline trientine tetrahydrochloride were prepared by blending about 300 mg of trientine tetrahydrochloride polymorphic Form A as the active ingredient with different amounts of colloidal anhydrous silica, crospovidone, glycerol dibehenate, magnesium aluminosilicate, mannitol, microcrystalline cellulose, magnesium stearate, stearic acid or/and talc as pharmaceutically acceptable excipients using a Kalweka All Purpose Equipment (Model: HD-410AC) mixing device (available from Rimek) at a mixing speed of 20 rpm for 15 minutes.

The mixtures obtained thereby were subjected to direct compression in a 16 Station Rotary Tablet Machine (Model : CMD3-16) tablet press (available from Cadmach) at a compression force of 29.4 kN (3 tf) for a compression time of less than 2 seconds, so as to obtain core tablets having a weight of 612 mg. The core tablets were finally coated in Ganscoater (Model: GAC250) coating machine (available from Gansons) with 18 mg of a polyvinyl alcohol-based immediate release coating film, so as to prepare film-coated tablets each having a total weight of 630 mg. Physicochemical properties of the respective core tablets and film-coated tablets are shown in Tables 1 to 3.

**Table 1**

| Tablet batch number | | Example 1 | Example 2 |
|---|---|---|---|
| Tablet batch size | | 500 tablets | 500 tablets |
| Tablet manufacturing process | | Direct compression | Direct compression |
| Type of punches used in tablet manufacturing process | | 18.2 × 8.8 mm plain oval shaped, shallow concave punches. Material of construction: PK3 for punches & tungsten carbide both sides taper dies | 18.2 × 8.8 mm plain oval shaped, shallow concave punches. Material of construction: PK3 for punches & tungsten carbide both sides taper dies |
| Polymorphic form of trientine tetrahydrochloride | | Form A | Form A |
| Particle size distribution of crystalline trientine tetrahydrochloride [µm] | | d10: 15.20 | d10: 15.20 |
| | | d50: 131.0 | d50: 131.0 |
| | | d90: 311.0 | d90: 312.0 |
| Measurement method of particle size distribution | | Dry method | Dry method |
| Formulation of core tablet [mg] | Trientine tetrahydrochloride | 312.05 | 312.05 |
| | Mannitol (Pearlitol^{®} SD 200) | 104.95 | 104.95 |
| | Magnesium aluminometasilicate (Neusilin^{®} US2) | 150.00 | 150.00 |
| | Crospovidone (Polyplasdone^{®} XL) | 20.00 | 20.00 |
| | Stearic acid | --- | 25.00 |
| | Glycerol dibehenate | 25.00 | --- |
| Weight of core tablet [mg] | | 612.00 | 612.00 |
| Hardness of core tablet [N] | | 114 to 133 | 152 to 156 |
| Formulation of tablet coating [mg] | PVA-based immediate release coating film (Opadry^{®} AMB II) | 18.00 | 18.00 |
| Weight of coated tablet [mg] | | 630.00 | 630.00 |
| Evaluation of core tablet | Stickiness of core tablet mixture | Sticking after compression of 50 tablets | Low sticking as compared to Example 1 |
| | Flowability of core tablet mixture | Not determined | Not determined |

**Table 2**

| Tablet batch number | | Example 3 | Example 4 |
|---|---|---|---|
| Tablet batch size | | 1000 tablets | 1000 tablets |
| Tablet manufacturing process | | Direct compression | Direct compression |
| Type of punches used in tablet manufacturing process | | 7.2 × 8.2 mm oval shaped shallow concave plain punches. MOC: S7 | 7.2 × 8.2 mm oval shaped shallow concave plain punches. MOC: S7 |
| Polymorphic form of trientine tetrahydrochloride | | Form A | Form A |
| Particle size distribution of crystalline trientine tetrahydrochloride [µm] | | d10: 21.31 | d10: 21.31 |
| | | d50: 67.70 | d50: 67.70 |
| | | d90: 131.27 | d90: 131.29 |
| Measurement method of particle size distribution | | Wet method | Wet method |
| Formulation of core tablet [mg] | Trientine tetrahydrochloride | 300.00 | 300.00 |
| | Mannitol (Pearlitol^{®} SD 200) | --- | 100.00 |
| | Colloidal anhydrous silica | 6.00 | 6.00 |
| | Crospovidone (Polyplasdone^{®} XL) | 10.00 | 10.00 |
| | Microcrystalline cellulose (Avicel^{®} PH 200 / Vivapur^{®} 102 SCG) | 261.00 | 161.00 |
| | Stearic acid | 25.00 | 25.00 |
| | Talc | 10.00 | 10.00 |
| Weight of core tablet [mg] | | 612.00 | 612.00 |
| Hardness of core tablet [N] | | 120 to 128 | 124 to 138 |
| Formulation of tablet coating [mg] | PVA-based immediate release coating film (Opadry^{®} AMB II) | 18.00 | 18.00 |
| Weight of coated tablet [mg] | | 630.00 | 630.00 |
| Hardness of coated tablet [N] | | 159 to 175 | 166 to 175 |
| Evaluation of core tablet | Stickiness of core tablet mixture | No sticking to production equipment | Die wall sticking after compression |
| | Flowability of core tablet mixture | Good | Good |

**Table 3**

| Tablet batch number | | Example 5 | Example 6 |
|---|---|---|---|
| Tablet batch size | | 1500 tablets | 1500 tablets |
| Tablet manufacturing process | | Direct compression | Direct compression |
| Type of punches used in tablet manufacturing process | | Compressed with 16.00 × 8.00 mm capsule shaped shallow concave punches with breaklines on upper and lower punch. MOC: PK3 | Compressed with 16.00 × 8.00 mm capsule shaped shallow concave punches with breaklines on upper and lower punch. MOC: PK3 |
| Polymorphic form of trientine tetrahydrochloride | | Form A | Form A |
| Particle size distribution of crystalline trientine tetrahydrochloride [µm] | | d10: 21.31 | d10: 7.64 |
| | | d50: 67.70 | d50: 28.96 |
| | | d90: 131.39 | d90: 63.34 |
| Measurement method of particle size distribution | | Wet method | Wet method |
| Formulation of core tablet [mg] | Trientine tetrahydrochloride | 300.00 | 300.39 |
| | Mannitol (Pearlitol^{®} SD 200) | 300.00 | 299.61 |
| | Colloidal anhydrous silica | 6.00 | 6.00 |
| | Magnesium stearate | 6.00 | 6.00 |
| Weight of core tablet [mg] | | 612.00 | 612.00 |
| Hardness of core tablet [N] | | 137 to 150 | 149 to 164 |
| Formulation of tablet coating [mg] | PVA-based immediate release coating film (Opadry^{®} AMB II) | 18.00 | 18.00 |
| Weight of coated tablet [mg] | | 630.00 | 630.00 |
| Hardness of coated tablet [N] | | 190 to 208 | 222 to 244 |
| Evaluation of core tablet mixture | Stickiness of core tablet mixture | No sticking to production equipment | No sticking to production equipment |
| | Flowability of core tablet mixture | Good | Not good |

As can be deduced from Table 1, a mixture obtained by blending trientine tetrahydrochloride polymorphic Form A with a combination of mannitol, magnesium aluminometasilicate, crospovidone and stearic acid as pharmaceutically acceptable excipients has lower stickiness than a mixture obtained by blending trientine tetrahydrochloride polymorphic Form A with a combination of mannitol, magnesium aluminometasilicate, crospovidone and glycerol dibehenate.

Table 2 shows that a mixture obtained by blending trientine tetrahydrochloride polymorphic Form A with a combination of colloidal anhydrous silica, crospovidone, microcrystalline cellulose, stearic acid and talc as pharmaceutically acceptable excipients has lower stickiness than a mixture obtained by blending trientine tetrahydrochloride polymorphic Form A with a combination of mannitol, colloidal anhydrous silica, crospovidone, microcrystalline cellulose, stearic acid and talc.

Table 3 provides evidence that a mixture obtained by blending crystalline trientine tetrahydrochloride realizing a d10 value of 21.31 µm, a d50 value of 67.70 µm and a d90 value of 131.39 µm with a combination of mannitol, colloidal anhydrous silica and magnesium stearate as pharmaceutically acceptable excipients has better flowability than a mixture obtained by blending crystalline trientine tetrahydrochloride realizing a d10 value of 7.64 µm, a d50 value of 28.96 µm and a d90 value of 63.34 µm with the same combination of excipients.

Hence, omission of glycerol dibehenate and mannitol in a mixture underlying a film-coated tablet of crystalline trientine tetrahydrochloride can improve handling of the mixture to be tableted during preparation of the pharmaceutical dosage form. In addition, the experimental data suggests that the use of crystalline trientine tetrahydrochloride having a particle size distribution in which the d10 value is at least 8 µm, the d50 value is at least 30 µm or/and the d90 value is at least 70 µm can improve handling of the mixture to be tableted, too.

### Example 7

A batch of film-coated tablets (batch size: 1500 tablets) comprising crystalline trientine tetrahydrochloride was prepared by blending 300.57 mg of trientine tetrahydrochloride polymorphic Form A as the active ingredient with 6.00 mg of colloidal anhydrous silica, 10.00 mg of crospovidone, 260.43 mg of microcrystalline cellulose, 25.00 mg of stearic acid and 10.00 mg of talc as pharmaceutically acceptable excipients using a Kalweka All Purpose Equipment (Model : HD-410AC) mixing device (available from Rimek) at a mixing speed of 20 rpm for 15 minutes.

The mixture obtained thereby was subjected to direct compression in a 16 Station Rotary Tablet Machine (Model: CMD3-16) tablet press (available from Cadmach) at different compression forces between 9.8 kN (1 tf) to 49.0 kN (5 tf) for a compression time of between 2 to 30 seconds, so as to obtain core tablets having a weight of 612 mg. The core tablets were finally coated in Ganscoater (Model: GAC250) coating machine (available from Gansons) with 18 mg of a TiO₂-free immediate release coating film, so as to prepare film-coated tablets each having a total weight of 630 mg. Physicochemical properties of the respective core tablets and film-coated tablets are shown in Table 4.

**Table 4**

| Tablet batch number | | Example 7 |
|---|---|---|
| Tablet batch size | | 1500 tablets |
| Tablet manufacturing process | | Direct compression |
| Type of punches used in tablet manufacturing process | | Compressed with 16.00 × 8.00 mm capsule shaped shallow concave punches with breaklines on upper and lower punch. MOC: PK3 |
| Polymorphic form of trientine tetrahydrochloride | | Form A |
| Particle size distribution of crystalline trientine tetrahydrochloride [µm] | | d10: 14.09 |
| | | d50: 57.47 |
| | | d90: 120.10 |
| Measurement method of particle size distribution | | Wet method |
| Formulation of core tablet [mg] | Trientine tetrahydrochloride | 300.57 |
| | Colloidal anhydrous silica | 6.00 |
| | Crospovidone (Polyplasdone^{®} XL) | 10.00 |
| | Microcrystalline cellulose (Avicel^{®} PH 200 / Vivapur^{®} 102 SCG) | 260.43 |
| | Stearic acid | 25.00 |
| | Talc | 10.00 |
| Weight of core tablet [mg] | | 612.00 |
| Hardness of core tablet [N] | | 140 to 200 |
| Formulation of tablet coating [mg] | TiO₂-free immediate release coating film (Opadry^{®} TF) | 18.00 |
| Weight of coated tablet [mg] | | 630.00 |
| Evaluation of core tablet | Stickiness of core tablet mixture | No sticking to production equipment |
| | Flowability of core tablet mixture | Good |

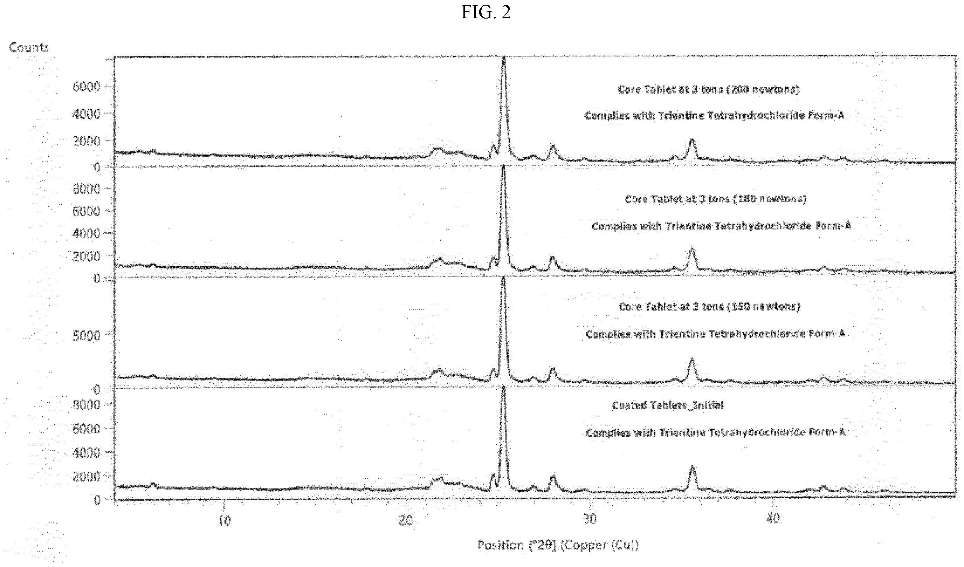

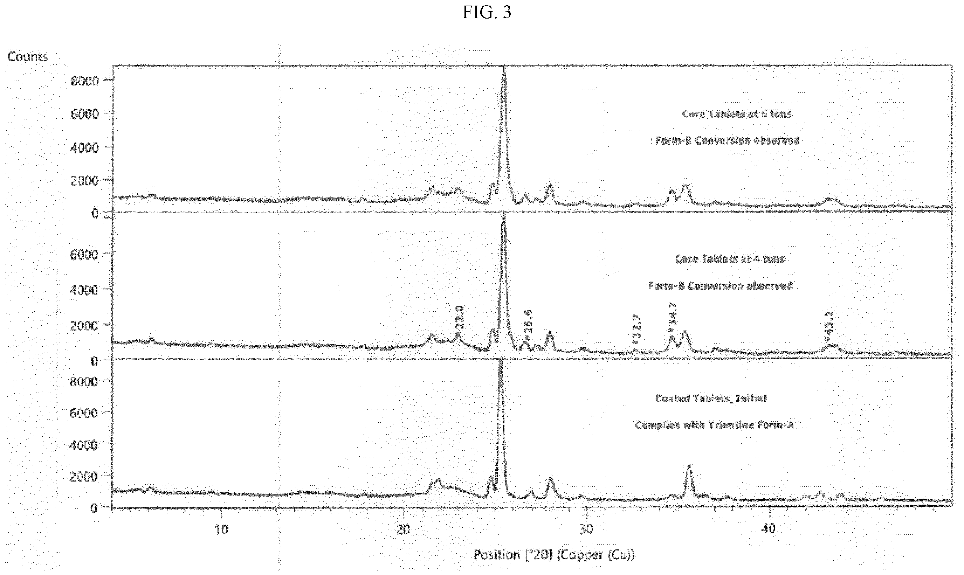

As can be derived from Figs. 1 and 2, application of a compression force of 9.8 kN (1 tf) or 29.4 kN (3 tf) for less than 2 seconds to a core tablet comprising trientine tetrahydrochloride Form A having a hardness of 140 to 200 N according to Example 7 does not cause any conversion from trientine tetrahydrochloride polymorphic Form A to trientine tetrahydrochloride polymorphic Form B. On the other hand, Fig. 3 shows that, at a compression force of 39.2 kN (4 tf) or 49.0 kN (5 tf), conversion from polymorphic Form A to polymorphic Form B starts. Similarly, Fig. 4 reveals that compression of the mixture underlying the core tablet at a compression force of 9.8 kN (1 tf) or 49.0 kN (5 tf) for 30 seconds also causes conversion from polymorphic Form A to polymorphic Form B.

Since trientine tetrahydrochloride polymorphic Form A is sensitive to compression stress, the stability of the active ingredient in a tablet can be increased by controlling the hardness of the tablet at less than 300 N, controlling the compression force at less than 39.2 kN (4 tf), or/and controlling the compression time at less than 30 seconds. In particular, conversion of trientine tetrahydrochloride polymorphic Form A to trientine tetrahydrochloride polymorphic Form B can be reliably prevented by controlling the compression time during direct compression of the mixture to be tableted at 2 seconds or less, thereby increasing stability of the active ingredient in the pharmaceutical dosage form.

## Claims

1. Pharmaceutical dosage form comprising crystalline trientine tetrahydrochloride and at least one pharmaceutically acceptable excipient.

2. Pharmaceutical dosage form of claim 1, wherein the crystalline trientine tetrahydrochloride has an average particle size (d50 value) of 30 to 600 µm, as determined in accordance with the wet method disclosed in the description.

3. Pharmaceutical dosage form of claim 1 or 2, wherein the crystalline trientine tetrahydrochloride has a d90 value of 70 to 1000 µm, as determined in accordance with the wet method disclosed in the description.

4. Pharmaceutical dosage form of any one of claims 1 to 3, wherein the crystalline trientine tetrahydrochloride has a d10 value of 8 to 500 µm, as determined in accordance with the wet method disclosed in the description.

5. Pharmaceutical dosage form of any one of claims 1 to 4, wherein the at least one pharmaceutically acceptable excipient does not include mannitol.

6. Pharmaceutical dosage form of any one of claims 1 to 5, wherein the at least one pharmaceutically acceptable excipient includes microcrystalline cellulose.

7. Pharmaceutical dosage form of any one of claims 1 to 6, wherein the at least one pharmaceutically acceptable excipient does not include glycerol dibehenate.

8. Pharmaceutical dosage form of any one of claims 1 to 7, wherein the at least one pharmaceutically acceptable excipient includes a C1 to C30 fatty acid.

9. Pharmaceutical dosage form of any one of claims 1 to 8, wherein the pharmaceutical dosage form is a tablet.

10. Pharmaceutical dosage form of claim 9, wherein the tablet has a hardness of less than 300 N, as determined in accordance with the method disclosed in the description.

11. Pharmaceutical dosage form of any one of claims 1 to 10, wherein crystalline trientine tetrahydrochloride is present in Form A **characterised by** an XRPD pattern having peaks at 21.8, 25.2, 26.9, 28.2 and 35.7 ± 0.1°2θ.

12. Pharmaceutical dosage form of any one of claims 1 to 11, wherein the dosage form does not contain any detectable amounts of trientine tetrahydrochloride Form B **characterised by** an XRPD pattern having at least two peaks selected from the peaks at 22.9, 25.4, 25.8, 26.6, 34.6 and 35.3 ± 0.1°2θ.

13. Process of preparing a pharmaceutical dosage form according to any one of claims 1 to 12, the process comprising the steps of:
(i) providing crystalline trientine tetrahydrochloride and at least one pharmaceutically acceptable excipient;
(ii) blending the components from step (i) to obtain a mixture;
(iii) direct compressing the mixture of step (ii) to obtain a tablet;
(iv) optionally coating the tablet.

14. Process of preparing a pharmaceutical dosage form according to claim 13, wherein the direct compression of step (iii) is carried out using tungsten carbide dies and PK3 steel punches.

15. Process of preparing a pharmaceutical dosage form according to claim 13 or 14, wherein the compression force applied in step (iii) is less than 39.2 kN (4 tf) for 2 seconds or less.
